# EUROPEAN PATENT APPLICATION

(11) **EP 1 621 134 A1**
(43) Date of publication of application: **01.02.2006**
(21) Application number: 04721998.5
(22) Date of filing: 19.03.2004
(51) Int. Cl.: A61B 8/00

(54) **ULTRASONIC PROBE AND ULTRASONOGRAPHIC DEVICE**

(30) Priority: 20.03.2003 JP 2003078833
(71) Applicant: MATSUSHITA ELECTRIC INDUSTRIAL CO., LTD., Kadoma-shi, Osaka 571-8501 (JP)
(72) Inventor: AKIYAMA, Hisashi, . (JP); FUJII, Kiyoshi, . (JP)
(74) Representative: Schorr, Frank Jürgen
(86) International application number: PCT/JP2004/003745
(87) International publication number: WO 2004/082482

(57) **Abstract**

An excellent ultrasonic diagnostic apparatus is provided that can form a three-dimensional image in a more spatially-correct position regardless of an ultrasonic probe to be used, without leading to deterioration of the productivity of the treatment. An ultrasonic probe includes an ultrasonic transducer, a rotation transmission mechanism, a rotary encoder, a transducer-swinging motor and an encoder correction ROM. In the encoder correction ROM, an actual swing scanning angle of the ultrasonic transducer with respect to each count value that is obtained by counting pulses from the rotary encoder is stored in advance. A three-dimensional image processing means forms a three-dimensional image of a principal cross-section scanning plane in a direction at the actual swing scanning angle, while correcting an encoder count value from an encoder counter according to contents of the encoder correction ROM that are read out by a main controlling means.

## Description

### Technical Field

The present invention relates to an ultrasonic probe that is directed to obtaining three-dimensional echo data by receiving an ultrasonic echo that is obtained by the reflection of an ultrasonic beam transmitted with respect to a tissue in a living body or the like, and relates to an ultrasonic diagnostic apparatus in which the ultrasonic probe is used.

### Background Art

Conventionally, as an ultrasonic probe for obtaining three-dimensional data, which is used in an ultrasonic diagnostic apparatus intended to display the condition of a tissue in a living body three-dimensionally, an ultrasonic probe including an ultrasonic transducer for scanning an ultrasonic beam, which is configured so as to swing mechanically the ultrasonic transducer to perform swing scanning in a direction that crosses the beam scanning direction, is known (see, for example, JP 3(1991)-184532 A). By performing ultrasonic beam scanning (hereinafter, called principal cross-section scanning) and swing scanning at the same time, echo data corresponding to a line of intersection of both scanning planes that move momentarily, that is, three-dimensional echo data, can be obtained.

The obtained three-dimensional echo data is subjected to three-dimensional image processing, thereby displaying an image within a plane as if it has a depth, displaying its required cross section or the like.

For such three-dimensional image processing, a directional component of each echo data in a three-dimensional space must be known.

In the case where the ultrasonic transducer is composed of a plurality of element arrays, beam scanning is performed electronically, and thus a directional component of the echo data within a beam scanning plane can be obtained by a scanning position, an arrangement of the transducer elements composing the ultrasonic transducer and a beam direction.

Whereas, the ultrasonic probe is configured so that an angle of the swing scanned plane being scanned mechanically is obtained by counting pulses from a rotary encoder that is provided to a rotation axis of a motor for swinging the ultrasonic transducer.

Recently, a three-dimensional image formed by an ultrasonic diagnostic apparatus has been used for observing a condition of a tissue in a living body, penetrating while monitoring the three-dimensional image and a guideline, or measuring a distance and an angle of an organ, a tumor, a fetus or the like, thus enhancing the usability thereof.

In the light of the requirements for such medical purposes, it is necessary for an ultrasonic diagnostic apparatus to form a three-dimensional image with higher precision than conventional ultrasonic diagnostic apparatuses, that is, to form an image in a spatially correct position.

However, in the above-mentioned conventional ultrasonic diagnostic apparatus, a swing scanning angle that is necessary for forming a three-dimensional image is obtained only by counting pulses output from a rotary encoder that is provided on a rotation axis of a swinging motor in an ultrasonic probe. Generally, in a mechanical scanning using a motor, an actual swing scanning angle of an ultrasonic transducer with respect to each count value that is obtained by counting pulses from a rotary encoder varies according to the individual ultrasonic probe, due to a variation in installation precision between a rotation axis of a motor and a rotary encoder, wobbling of a rotation transmission mechanism, a variation in installation precision between an ultrasonic transducer and the rotation transmission mechanism, a variation of the rotary encoder itself or the like.

For example, an ultrasonic probe that corrects, based on encoder information, a scanning error (flexure or elongation of a rotation transmission mechanism with respect to a speed) occurring according to a change in amount of a scanning load of the ultrasonic probe is known (see, for example, JP 2(1990)-57242 A). However, this kind of ultrasonic probe requires to have a power supply, a switch and the like for the correction and needs a discontinuance of the operation thereof, which leads to an increase in the size of an ultrasonic diagnostic apparatus and causes a problem in workability and cost.

Even in the case where the main unit of the ultrasonic diagnostic apparatus has a means of correcting the above-stated variations, it is necessary to perform a correction process, such as a process of inputting an actual swing scanning angle of the ultrasonic transducer with respect to each count value, every time when the used ultrasonic probe is changed.

Also, in the case of obtaining three-dimensional data by reciprocation of the swing scanning, the actual swing scanning angle of the ultrasonic transducer with respect to each count value that is obtained by counting the pulses from the rotary encoder may differ between its forward path and its return path, which is caused by wobbling of the rotation transmission mechanism or the like.

That is, the swing scanning angle with respect to each count value varies according to the individual ultrasonic probe, and differs between the forward path of the swinging and the return path thereof. Therefore, according to the ultrasonic probe to be used, the formed three-dimensional image is distorted, is displaced, flickers due to the reciprocation of its swinging or the like, which may result in problems such as penetrating in a direction deviated from the direction expected by the operator, and a large error in a result of the measurement of a distance and an angle.

Moreover, if it is intended to correct these variations for each ultrasonic probe to be used, for solving these problems, the operator has to perform a correction process every time when the ultrasonic probe is changed, which causes deterioration in the productivity of the treatment.

In this connection, for example, an ultrasonic diagnostic apparatus that performs correction by adding a backlash correction signal to an output signal of an encoder in advance is known (see JP 1(1989)-227743 A). However, this apparatus has a problem that only a fixed deviation of the swing scanning angle in a swinging reciprocation can be corrected.

### Disclosure of Invention

The present invention intends to solve the above-mentioned conventional problems so as to provide an excellent ultrasonic diagnostic apparatus that can form a three-dimensional image in a more spatially-correct position regardless of an ultrasonic probe to be used, without leading to deterioration of the productivity of the treatment, and to provide an ultrasonic probe that is suitable for being applied in such ultrasonic diagnostic apparatus.

In order to attain the above-mentioned object, the ultrasonic probe according to the present invention includes: an ultrasonic transducer that scans an ultrasonic beam; a transducer-swinging motor that allows the ultrasonic transducer to perform swing scanning in a direction crossing a scanning direction of the ultrasonic beam; a rotary encoder that generates a pulse according to a rotational position of the transducer-swinging motor; and an encoder correction ROM that stores an actual swing scanning angle of the ultrasonic transducer with respect to each count value obtained by counting pulses from the rotary encoder, and outputs the stored actual swing scanning angle of the ultrasonic transducer to outside.

According to this configuration, the actual swing scanning angle of the ultrasonic transducer with respect to each count value that is obtained by counting the pulses from the rotary encoder can be stored in an encoder correction ROM in advance, and the actual swing scanning angle of the ultrasonic transducer with respect to each count value, which varies according to the individual ultrasonic probe, depending on a method of mechanical scanning, can be known.

Moreover, in the ultrasonic probe according to the present invention, the encoder correction ROM preferably stores swing directional angles that are different between a forward path of swing scanning and a return path of the swing scanning.

According to this configuration, the actual swing scanning angles of the ultrasonic transducer on the forward path of the swinging and on the return path thereof with respect to the count value that is obtained by counting the pulses from the rotary encoder can be stored in the encoder correction ROM in advance, and the actual swing scanning angle of the ultrasonic transducer with respect to each count value, which varies according to the individual ultrasonic probe, depending on a method of mechanical scanning, and differs between the forward path of the swinging and the return path thereof, can be obtained.

Moreover, in order to attain the above-mentioned object, a first ultrasonic diagnostic apparatus according to the present invention includes : the ultrasonic probe according to the present invention; a transmitting/receiving means that excites vibrators of the ultrasonic transducer and receives an ultrasonic echo reflected by a subject; an encoder counter that counts pulses from the rotary encoder; a main controlling means that reads out, from the encoder correction ROM in the ultrasonic probe, the actual swing scanning angle of the ultrasonic transducer with respect to each of the counter value; a motor controlling means that performs driving control on the transducer-swinging motor according to the count value from the encoder counter; a three-dimensional image processing means that forms a three-dimensional image based on ultrasonic echo data obtained by the transmitting/receiving means, the count value from the encoder counter and the actual swing scanning angle of the ultrasonic transducer with respect to each of the count value that is provided by the main controlling means; and an image display means that displays the three-dimensional image.

According to this configuration, a three-dimensional image can be formed while being corrected, based on the actual swing scanning angle of the ultrasonic transducer with respect to each count value, which varies according to the individual ultrasonic probe.

In order to attain the above-mentioned object, a second ultrasonic diagnostic apparatus according to the present invention includes: the ultrasonic probe according to the present invention; a transmitting/receiving means that excites vibrators of the ultrasonic transducer and receives an ultrasonic echo reflected by a subject; an encoder counter that counts pulses from the rotary encoder; a main controlling means that reads out, from the encoder correction ROM in the ultrasonic probe, the actual swing scanning angle of the ultrasonic transducer with respect to each of the count value; a motor controlling means that performs driving control on the transducer-swinging motor according to the count value from the encoder counter and the actual swing scanning angle of the ultrasonic transducer with respect to each of the count value that is provided by the main controlling means; a three-dimensional image processing means that forms a three-dimensional image based on ultrasonic echo data obtained by the transmitting/receiving means; and an image display means that displays the three-dimensional image.

According to this configuration, the swinging can be controlled while a swing scanning direction is corrected based on the actual swing scanning angle of the ultrasonic transducer with respect to each count value, which varies according to the individual ultrasonic probe.

According to the above-mentioned configuration, an excellent ultrasonic diagnostic apparatus that can form a three-dimensional image in a more spatially-correct position regardless of the ultrasonic probe to be used, without leading to deterioration of the productivity of the treatment, can be obtained.

Moreover, a low-cost and small-sized ultrasonic probe can be realized by using a flash ROM or an E-square ROM that is available at low cost and small in size as the encoder correction ROM.

In addition, since correction data with nonvolatility is stored in the encoder correction ROM in advance, time for obtaining data that is necessary for the correction is not required additionally.

Furthermore, unlike the conventional example, deviation of the angle between the forward path of the swinging and the return path thereof can be corrected flexibly according to the swing scanning angle (that is, an output value of the encoder) of the ultrasonic transducer.

### Brief Description of Drawings

FIG. 1 is a block diagram showing an example of a configuration of the ultrasonic diagnostic apparatus according to the respective embodiments of the present invention.
FIG. 2 is a view showing a configuration of the rotary encoder 4 of FIG. 1, and waveforms of a Z-pulse and an A-pulse obtained thereby.
FIG. 3 is a view showing contents stored in the encoder correction ROM 9 of FIG. 1.
FIG. 4 is a view showing a state of forming a three-dimensional image in the embodiments of the present invention.
FIG. 5 is a view showing contents of an encoder correction ROM that stores different encoder correction values between for the forward path of the swing scanning and for the return path thereof in the embodiments of the present invention.
FIG. 6 is a view showing a state of forming a three-dimensional image obtained by respectively correcting swing scanning angles on the forward path of the swing scanning and on the return path thereof in the embodiments of the present invention.

### Description of the Invention

Preferred embodiments of the present invention will be described below with reference to the drawings.

### (Embodiment 1)

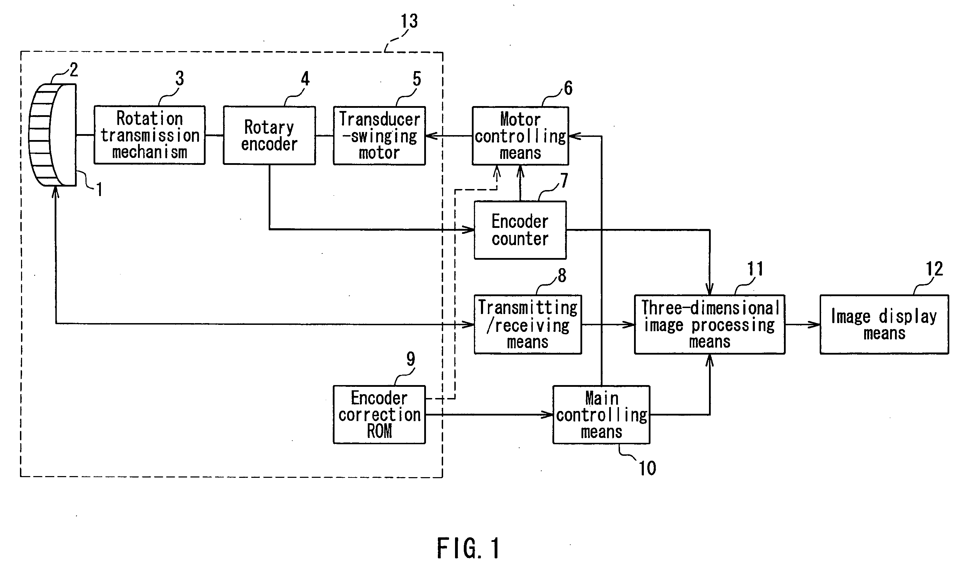

FIG. 1 is a block diagram showing an example of a configuration of the ultrasonic diagnostic apparatus according to Embodiment 1 of the present invention. The ultrasonic diagnostic apparatus shown in FIG. 1 will be applied also in the respective embodiments described below.

In FIG. 1, the ultrasonic diagnostic apparatus according to the present embodiment includes an ultrasonic transducer 1 with a plurality of transducer elements 2 arranged in array, the transducer element 2 transmitting an ultrasonic beam into a living body and converting an ultrasonic echo from a tissue in the living body into an electric signal. Each of the transducer elements 2 is excited by a transmission pulse provided by a transmitting/receiving means 8. The transmitting/receiving means 8 is controlled to provide the transmission pulses with different phases to a part of or all of the transducer elements 2 arranged in the ultrasonic transducer 1, so that the transmission pulse is focused at a predetermined depth in the living body, that is, a transmission beam is formed.

The ultrasonic beam transmitted to the living body as mentioned above returns as an echo from the respective tissues in the living body momentarily. The transmitting/receiving means 8 performs an adding operation with respect to the ultrasonic echoes that are converted into the electric signals by the transducer elements 2 of the ultrasonic transducer 1 so that each reception beam is formed in the predetermined direction, after providing different delay times with respect to reception signals from transducer elements 2. The above-described transmission beam and this reception beam form one acoustic scanning line by a transmission/reception, that is, the transmitting/receiving means 8 generates and outputs ultrasonic echo data along this acoustic scanning line.

As mentioned above, the transmission/reception is performed so as to form acoustic scanning lines in different directions by switching a group of the transducer elements to be used for the transmission/reception sequentially, or changing directions of the transmission/reception beams. As a result, one principal cross-section scanning plane is formed.

Moreover, the ultrasonic diagnostic apparatus according to the present embodiment includes a transducer-swinging motor 5 that allows the ultrasonic transducer 1 to perform swing scanning in a direction crossing the above-mentioned principal cross-section scanning plane, and the transducer-swinging motor 5 is subjected to driving control by a motor controlling means 6. By performing a principal cross-section scanning and a swing scanning at the same time, the transmitting/receiving means 8 can generate the ultrasonic echo data that corresponds to a line of intersection of the principal cross-section scanning plane and a swing scanning plane. Generally, both of the scanning planes are not scanned independently, but are scanned so as to obtain ultrasonic echo data of a certain three-dimensional part in an organic body equally. That is, the principal cross-section scanning and the swing scanning are performed so that the number of the principal cross-section scanning planes per one swing scanning is always constant, and angles between the respective principal cross-section scanning planes are substantially the same.

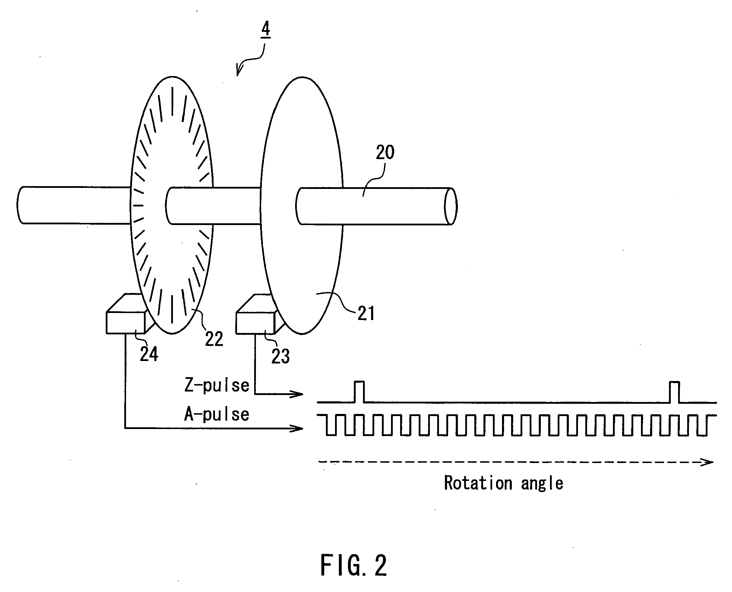

Therefore, the motor controlling means 6 needs to control the swinging while constantly monitoring a swing scanning angle of the ultrasonic transducer 1 that is connected to the transducer-swinging motor 5. For obtaining this swing scanning angle, the ultrasonic diagnostic apparatus according to the present embodiment includes a rotary encoder 4 that is provided on a rotation axis of the transducer-swinging motor 5. A preferred example of the rotary encoder 4 is shown in FIG. 2. This rotary encoder 4 includes a Z-pulse rotor 21 provided on a rotation axis 20 so that one pulse (hereinafter, called a Z-pulse) is output at a certain angle of the rotation axis 20. For example, in a magnetic encoder, the Z-pulse rotor 21 is magnetized so as to generate one Z-pulse per one rotation, and a Z-pulse sensor 23 detects the magnetized part of the Z-pulse rotor 21 and outputs a Z-pulse. Similarly, the rotary encoder 4 includes an A-pulse rotor 22 and an A-pulse sensor 24 so as to generate several hundreds of pulses (hereinafter, called A-pulses) fixedly per one rotation of the rotation axis 20.

The magnetic encoder was exemplified in the above description, but an optical encoder or a mechanical encoder may be applied in the configuration of the present invention.

An encoder counter 7 is reset by the Z-pulses from the rotary encoder 4, and is counted up or down by the A-pulses. And, a count value thereof corresponds to an angle of the rotation axis of the transducer-swinging motor 5, that is, the swing scanning angle of the ultrasonic transducer 1. The motor controlling means 6 can obtain the current swing scanning angle of the ultrasonic transducer 1 according to the count value provided by the encoder counter 7, as mentioned above, and thus can control the transducer-swinging motor 5 so as to move the ultrasonic transducer 1 to a subsequent predetermined swing scanning angle.

The ultrasonic echo data on the certain three-dimensional part in the living body, which is obtained by performing the principal cross-section scanning and the swing scanning in synchronization as mentioned above, is transmitted from the transmitting/receiving means 8 to a three-dimensional image processing means 11. In the three-dimensional image processing means 11, a three-dimensional image processing is performed with respect to the obtained ultrasonic echo data so that a configuration of the certain three-dimensional part in the living body may be displayed as if it has a depth, or the configuration viewed from a point of view in any directions may be displayed on an image display means 12 having a flat or slowly curved display surface. For performing this three-dimensional image processing, a directional component of the obtained ultrasonic echo data of each acoustic scanning line in a three-dimensional space is required to be known. The three-dimensional image processing means 11 obtains a directional component of an angle of the scanning direction on the principal cross-section scanning plane, by an arrangement of the transducer elements 2 composing the ultrasonic transducer 1 and directions of the transmission/reception beams. Whereas, the three-dimensional image processing means 11 obtains a directional component of the angle of the scanning direction on the swing scanning plane by the count value provided by the encoder counter 7.

Generally in the ultrasonic diagnostic apparatus using a mechanical scanning method, the actual swing scanning angle of the ultrasonic transducer 1 with respect to each count value that is obtained by counting pulses from the rotary encoder 4 varies according to the ultrasonic diagnostic apparatus, due to an accumulation of respective variations of installation precision between the rotation axis of the transducer-swinging motor 5 and the rotary encoder 4, precision of a rotation transmission mechanism 3 with respect to the rotation axis of the transducer-swinging motor 5 and the ultrasonic transducer 1, installation precision between the ultrasonic transducer 1 and the rotation transmission mechanism 3, precision of an angle of the rotary encoder 4 for generating the Z-pulse, a linearity of the number of A-pulses with respect to the angle of the rotation axis and the like.

The encoder correction ROM 9 is provided for storing the actual swing scanning angle of the ultrasonic transducer 1 with respect to each count value that is to be obtained from the encoder counter 7, or a numerical value corresponding to the swing scanning angle is stored. The encoder correction ROM 9 holds the stored value after an electric current in the ultrasonic diagnostic apparatus is OFF, and does not lose the value still after an electric current is ON again.

The value stored in the encoder correction ROM 9 is read by the main controlling means 10, and the value or a value corrected by an appropriate value subsequently is transmitted via the main controlling means 10 to the three-dimensional image processing means 11, the motor controlling means 6 or both of the three-dimensional image processing means 11 and the motor controlling means 6.

Generally in an ultrasonic diagnostic apparatus, the ultrasonic transducer 1 or the ultrasonic transducer 1 with its peripheral portions unified therewith can be separated from the main portion of the ultrasonic diagnostic apparatus, and is mobile, which is called an ultrasonic probe. That is, it is not necessary that a certain ultrasonic probe is always applied to the main portion of the same ultrasonic diagnostic apparatus.

In the ultrasonic diagnostic apparatus according to the present embodiment, the ultrasonic transducer 1, the rotary encoder 4, the transducer-swinging motor 5 and the encoder correction ROM 9 compose the ultrasonic probe 13, which can be separated from the main portion of the ultrasonic diagnostic apparatus. This is because, since substantially all portions that can cause the occurrence of the variations of the swing scanning angle with respect to the count value of the rotary encoder 4 are included, the encoder correction ROM 9 can correct the accumulation of the variations together, even in the case where the ultrasonic probe 13 is applied to a main portion of different ultrasonic diagnostic apparatus.

Accordingly, a configuration where other components of the ultrasonic diagnostic apparatus besides the above-described components are added in the ultrasonic probe 13 is, needless to say, possible as well.

### (Embodiment 2)

Next, Embodiment 2 of the present invention will be described with reference to FIG. 1 as well as Embodiment 1.

Generally, swing scanning mostly is performed as reciprocating scanning. This method is intended for forming a three-dimensional image at more real time. Also in this case, even when count values provided by the encoder counter 7 on a forward path of the swing scanning and on its return path are equal, real swing scanning angles thereof are often different, which is a problem caused by a mechanical scanning method, in addition to the above-mentioned problem.

This is caused mostly by the rotation transmission mechanism 3 that connects the rotation axis of the transducer-swinging motor 5 and the ultrasonic transducer 1. For example, in a rotation transmission mechanism using a gear, rotation angles of a passive gear with respect to an angle of a rotation axis of a motor in a forward rotation of the gear and in a reverse rotation thereof are different, due to the existence of backlash in portions where the gear and the passive gear are engaged with each other. Moreover, in the case of using a belt or a wire, a similar phenomenon occurs due to elongation of the belt or the wire.

In the case of forming the three-dimensional image by performing the swing scanning, if applying the count value provided by the encoder counter 7 directly to the three-dimensional image processing means 11, the above-mentioned phenomenon leads to a problem where positions of the three-dimensional image formed on the forward path and on the return path are different, that is, the three-dimensional image flickers, or a problem where distortions of the three-dimensional images formed on the forward path and on the return path are different.

In the ultrasonic diagnostic apparatus according to the present embodiment, since the encoder correction ROM 9 can store different correction data between the forward path of the swing scanning and the return path thereof, a correction data for the forward path can be used on the forward path of the swinging, and a correction data for the return path can be used on the return path thereof, thereby solving the above-described problem.

### (Embodiment 3)

Next, as Embodiment 3 of the present invention, an ultrasonic diagnostic apparatus that can form a more precise three-dimensional image by the ultrasonic probe 13 including the above-mentioned encoder correction ROM 9 and the like will be described with reference to FIGs. 2 to 6.

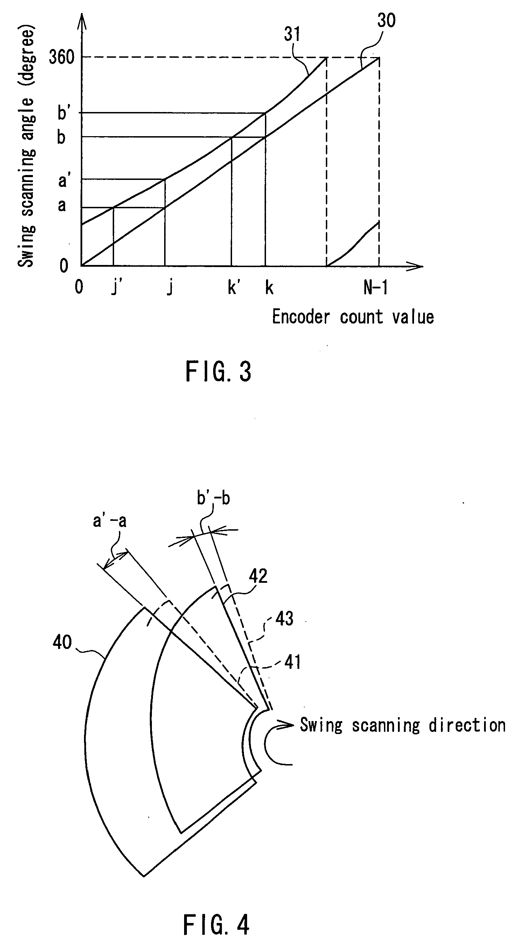

In the encoder correction ROM 9 of the ultrasonic diagnostic apparatus according to the present embodiment, a correction value 31 as shown in FIG. 3 is stored in advance. In FIG. 3, a straight line 30 shows a case where the swing scanning angle of the ultrasonic transducer 1 with respect to the count value of the encoder counter 7 is ideal, and where the encoder counter 7 is an up-counter, using the rotary encoder 4 that generates N number of A-pulses during one rotation of the rotation axis of the transducer-swinging motor 5 and a Z-pulse when the angle of the rotation axis is 0°. In addition, the present embodiment exemplifies the case where the angle of the rotation axis coincides with an angle of the ultrasonic transducer 1, but the case where a rotation speed transmission ratio of the rotation transmission mechanism 3 is not 1:1 also can be applied.

Whereas, the correction value 31 in FIG. 3 shows the actual swing scanning angle of the ultrasonic transducer 1 with respect to a count value provided by the encoder counter 7, and shows that, for example, when the count value is " j ", the actual angle for this ultrasonic probe is "a'" in spite of the ideal angle being " a ". Similarly, the actual angle is " b"' with respect to the count value of " k ", while the ideal angle is " b ".

From the encoder correction ROM 9 in which such a correction value 31 is stored, the correction value 31 is read out by the main controlling means 10, and is transmitted to the three-dimensional image processing means 11.

Next, a method by which the three-dimensional image processing means 11 forms a three-dimensional image using the correction value 31 will be described with reference to FIG. 4 in addition to FIG. 3. For example, when the count value provided by the encoder counter 7 is " j ", unless the correction value is provided, only a principal cross-section scanning plane 40 can be formed at the angle of " a " in the swing scanning direction in FIG. 4. On the other hand, since the actual angle of " a"' is obtained from the main controlling means 10 in advance, a principal cross-section scanning plane 41 can be formed in a direction deviated by the difference of " a'-a ". Similarly, a principal cross-section scanning plane 43 can be formed being shifted from a principal cross-section scanning plane 42, when the count value of " k " is obtained from the encoder counter 7.

As mentioned above, the three-dimensional image processing means 11 obtains the encoder correction value for the applied ultrasonic probe 13 in advance, and can form an image of the principal cross-section scanning plane at the actual swing scanning angle, while correcting the encoder count value with respect to the obtained ultrasonic echo data, thereby forming a more precise three-dimensional image of a tissue in a living body.

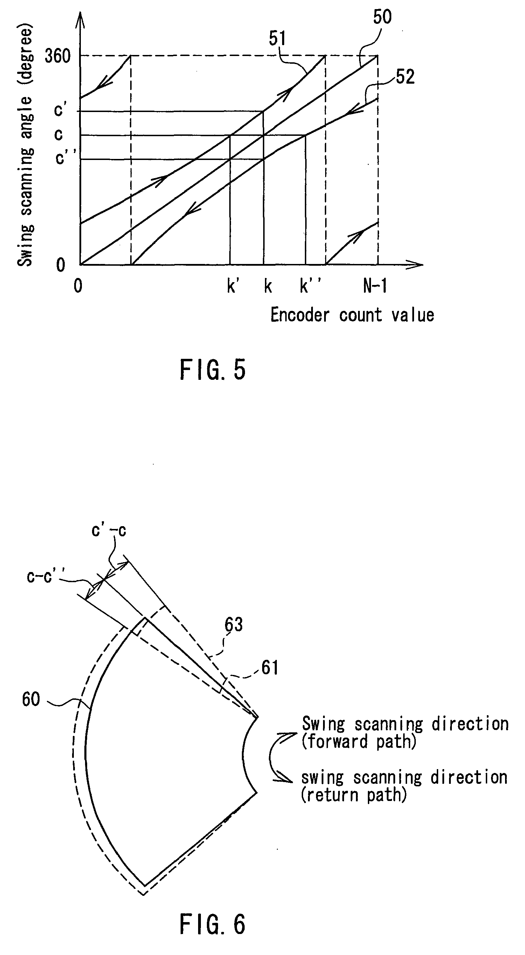

Moreover, an example of performing the swing scanning in reciprocation is shown in FIGs. 5 and 6. As shown in FIG. 5, in the encoder correction ROM 9, a forward-path correction values 51 having a direction of increasing the encoder count value and a return-path correction values 52 having a direction of decreasing the encoder count value are stored in advance. Ideally, the both correction values are on the same track as shown by a straight line 50. The encoder correction values 51 and 52 in FIG. 5 show that, for example, when the encoder count value is " k ", the actual swing scanning angle of the ultrasonic transducer on the forward path is " c' " and that on the return path is " c" ".

Since the forward-path correction value 51 and the return-path correction value 52 are transmitted to the three-dimensional image processing means 11 by the main controlling means 10 in advance, when the count value provided by the encoder counter 7 is " k ", unless the correction value is provided, only a principal cross-section scanning plane 60 can be formed at an angle of " c " in the swing scanning direction on both of the forward path and the return path, as shown in the example of FIG. 6. However, the three-dimensional image processing means 11 obtains the actual angle of "c"' on the forward path from the main controlling means 10 in advance, and thus forms an image of a principal-cross-section scanning plane 63 on the forward path in a direction deviated by the difference of " c'-c ", and forms an image of a principal cross-section scanning plane 61 on the return path in a direction deviated by the difference of "c-c" ".

As mentioned above, according to the ultrasonic diagnostic apparatus of the present invention, the three-dimensional image processing means 11 obtains the encoder correction values on both of the forward path and the return path of the swing scanning of the applied ultrasonic probe 13 in advance, and can form the image of the principal cross-section scanning plane at the actual swing scanning angle, while correcting the encoder count values on the forward path and the return path with respect to the obtained ultrasonic echo data to be different values, thereby forming a more precise three-dimensional image of a tissue in a living body.

### (Embodiment 4)

Next, as Embodiment 4 of the present invention, an ultrasonic diagnostic apparatus that can form a more precise three-dimensional image by the motor controlling means 6, using the encoder correction ROM 9, will be described.

In Embodiment 3, the three-dimensional image processing means 11 corrects the angle of the three-dimensional image to be formed, based on the encoder correction value, and the similar effect also can be obtained by the motor controlling means 6. As mentioned above, the correction value 31 in FIG. 3 is the actual swing scanning angle of the ultrasonic transducer 1 with respect to the count value provided by the encoder counter 7. This correction value is transmitted to the motor controlling means 6 by the main controlling means 10 in advance (shown as the arrow with the broken line in FIG. 1).

For example, when a desired swing scanning angle of the ultrasonic transducer 1 is " a ", unless the motor controlling means 6 has a correction value, there is no other means except controlling the transducer-swinging motor 5 so that the count value provided by the encoder counter 7 may be " j ". Here, since the actual swing scanning angle is " a"' when the count value is " j ", the ultrasonic transducer 1 is in the direction deviated from the desired value by " a'-a ". However, the motor controlling means 6 of the ultrasonic diagnostic apparatus according to the present invention obtains the encoder correction value in advance, and thus may control the transducer-swinging motor 5 so that the encoder count value may be " j'" with respect to the desired value of " a ".

If an angle of each principal cross-section scanning plane to be formed is provided by the main controlling means 10 to the three-dimensional image processing means 11 in advance, the three-dimensional image processing means 11 forms an image in a direction at the provided angle, because the obtained ultrasonic echo data is already corresponding to the principal cross-section scanning plane at the desired swing scanning angle. As a result, according to the ultrasonic diagnostic apparatus of the present embodiment, a three-dimensional image of a tissue in a living body can be formed more precisely.

As mentioned above, the present invention can provide an excellent ultrasonic diagnostic apparatus that can correct a variation in installation precision between the rotation axis of the motor and the rotary encoder, precision of the rotation transmission mechanism, a variation in installation precision between the ultrasonic transducer and the rotation transmission mechanism and a variation of the rotary encoder itself, which may vary according to the individual ultrasonic probe, and thus can form a three-dimensional image in a more spatially-correct position regardless of the ultrasonic probe to be used.

Also, since the corrections of the above-described variations are performed with respect to each ultrasonic probe, and the main portion of the ultrasonic diagnostic apparatus can obtain the correction data and correct the swing scanning angle of the ultrasonic transducer, an excellent ultrasonic diagnostic apparatus can be provided, which does not require the operator to perform any correction processes even when the applied probe is changed, and displays a three-dimensional image with high precision.

In addition, the encoder correction ROM can store swing directional angles that are different between the forward path of the swing scanning and the return path thereof, and thus can correct the actual swing scanning angles of the ultrasonic transducer with respect to the encoder count values, which are different between the forward path and the return path due to wobbling of the rotation transmission mechanism or the like, thereby providing an excellent ultrasonic diagnostic apparatus that can suppress a problem of flicker or distortion of the image depending on the swing reciprocation, regardless of the ultrasonic probe to be used.

Moreover, a low-cost and small-sized ultrasonic probe can be realized by using a flash ROM or an E-square ROM that is available at low cost and small in size as the encoder correction ROM.

Furthermore, since the correction data with nonvolatility is stored in the encoder correction ROM in advance, time for obtaining the data that is necessary for the correction is not required additionally

Still further, unlike the conventional example, deviation of the angle between the forward path of the swinging and the return path thereof can be corrected flexibly, according to the swing scanning angle (that is, the output value of the encoder) of the ultrasonic transducer.

## Claims

1. An ultrasonic probe, comprising:
an ultrasonic transducer that scans an ultrasonic beam;
a transducer-swinging motor that allows the ultrasonic transducer to perform swing scanning in a direction crossing a scanning direction of the ultrasonic beam;
a rotary encoder that generates a pulse according to a rotational position of the transducer-swinging motor; and
an encoder correction ROM that stores an actual swing scanning angle of the ultrasonic transducer with respect to each count value obtained by counting pulses from the rotary encoder, and outputs the stored actual swing scanning angle of the ultrasonic transducer to outside.

2. The ultrasonic probe according to Claim 1, wherein the encoder correction ROM stores swing directional angles that are different between a forward path of swing scanning and a return path of the swing scanning.

3. An ultrasonic diagnostic apparatus, comprising:
an ultrasonic probe comprising an ultrasonic transducer that scans an ultrasonic beam, a transducer-swinging motor that allows the ultrasonic transducer to perform swing scanning in a direction crossing a scanning direction of the ultrasonic beam, a rotary encoder that generates a pulse according to a rotational position of the transducer-swinging motor, and an encoder correction ROM that stores an actual swing scanning angle of the ultrasonic transducer with respect to each count value obtained by counting pulses from the rotary encoder, and outputs the stored actual swing scanning angle of the ultrasonic transducer to outside;
a transmitting/receiving means that excites vibrators of the ultrasonic transducer and receives an ultrasonic echo reflected by a subject;
an encoder counter that counts pulses from the rotary encoder;
a main controlling means that reads out, from the encoder correction ROM in the ultrasonic probe, the actual swing scanning angle of the ultrasonic transducer with respect to each of the counter value;
a motor controlling means that performs driving control on the transducer-swinging motor according to the count value from the encoder counter;
a three-dimensional image processing means that forms a three-dimensional image based on ultrasonic echo data obtained by the transmitting/receiving means, the count value from the encoder counter and the actual swing scanning angle of the ultrasonic transducer with respect to each of the count value that is provided by the main controlling means; and
an image display means that displays the three-dimensional image.

4. The ultrasonic diagnostic apparatus according to Claim 3, wherein the encoder correction ROM stores swing directional angles that are different between a forward path of swing scanning and a return path of the swing scanning.

5. An ultrasonic diagnostic apparatus, comprising:
an ultrasonic probe comprising an ultrasonic transducer that scans an ultrasonic beam, a transducer-swinging motor that allows the ultrasonic transducer to perform swing scanning in a direction crossing a scanning direction of the ultrasonic beam, a rotary encoder that generates a pulse according to a rotational position of the transducer-swinging motor, and an encoder correction ROM that stores an actual swing scanning angle of the ultrasonic transducer with respect to each count value obtained by counting pulses from the rotary encoder, and outputs the stored actual swing scanning angle of the transducer elements unit to outside;
a transmitting/receiving means that excites vibrators of the ultrasonic transducer and receives an ultrasonic echo reflected by a subject;
an encoder counter that counts pulses from the rotary encoder;
a main controlling means that reads out, from the encoder correction ROM in the ultrasonic probe, the actual swing scanning angle of the ultrasonic transducer with respect to each of the count value;
a motor controlling means that performs driving control on the transducer-swinging motor according to the count value from the encoder counter and the actual swing scanning angle of the ultrasonic transducer with respect to each of the count value that is provided by the main controlling means;
a three-dimensional image processing means that forms a three-dimensional image based on ultrasonic echo data obtained by the transmitting/receiving means; and
an image display means that displays the three-dimensional image.

6. The ultrasonic diagnostic apparatus according to Claim 5, wherein the encoder correction ROM stores swing directional angles that are different between a forward path of swing scanning and a return path of the swing scanning.
